# EUROPEAN PATENT SPECIFICATION

(11) **EP 0 888 547 B1**
(45) Date of publication and mention of the grant of the patent: **12.03.2003**
(21) Application number: 97907232.9
(22) Date of filing: 13.03.1997
(51) Int. Cl.: G01N 33/558

(54) **IMMUNOASSAY WITH WHOLE BLOOD**
IMMUNOASSAY VON VOLLBLUTPROBEN
ANALYSE IMMUNOLOGIQUE DU SANG ENTIER

(30) Priority: 14.03.1996 US 615132
(43) Date of publication of application: 07.01.1999
(73) Proprietor: SPECTRAL DIAGNOSTICS INC., Toronto, Ontario M9C 1C2 (CA)
(72) Inventor: BUNCE, Roger, Abraham, Birmingham B30 1DZ (GB); FREITAG, Helmut, E., D-69469 Weinheim (DE)
(74) Representative: Witz, Michael
(86) International application number: IB9700255
(87) International publication number: WO97034148

(56) References cited:
- EP-A- 0 269 240
- EP-A- 0 505 636
- EP-A- 0 585 914
- DE-C- 3 445 816
- DATABASE WPI Section Ch, Week 9419 Derwent Publications Ltd., London, GB; Class B04, AN 94-153815 XP002033689 & JP 06 094 718 A (DAIICHI RADIOISOTOPE KENKYUSHO) , 8 April 1994

## Description

### FIELD OF THE INVENTION

This invention relates to methods useful for diagnostic assays to determine the presence of analytes characteristic of a diseased condition such as cardiac malfunction or microbial infection. More particularly, it is useful for identifying analytes in whole blood, although it is not so limited.

### BACKGROUND OF THE INVENTION

A number of immunoassay procedures have recently been developed which utilize reactions taking place on a porous or cellular membrane, the reactions being detectable either visually or with an instrument such as a reflectometer. While not so limited, these procedures generally involve antigen/antibody reactions in which one member of the reactive pair is labelled with a detectable label. Typically, the label is an enzyme label or a sol label such as gold. The art is well aware of many useful labels and their method of operation. Hence, there is no need for further discussions of labels herein.

Typical immunochromatographic devices of this nature are described in several United States and foreign patents. For example, United States Patent 4,861,711 describes a device in which an analyte detected by antigen/antibody reactions which take place in a series of coplanar membranes in edge to edge contact.

United States Patents 4,477,575 and 4,816,224 describe laminar devices including a glass fiber layer for conducting similar reactions.

Other laminated devices are described in United States Patents:

| | |
|---|---|
| 4,774,192 | 5,079,142 |
| 4,753,776 | 5,096,809 |
| 4,933,092 | 5,110,724 |
| 4,987,065 | 5,144,890 |
| 5,075,078 | 5,290.678 |
| 5,135,716 | 5,591,645 |

U.S. Patent No. 5,135,716 utilizes an agglutinating agent to assist in the separation of red blood cells. All of the patents describe laminated devices.

So far as applicants are aware, no non-laminated immunochromatographic device operable with whole blood has been previously known or described.

Devices such as those described in the above identified patents are often difficult to manufacture because they are multi-layer and require several layers of porous and filtration strips to insure accurate results. For detection of analytes in whole blood, it is necessary to remove red blood cells so that they will not interfere with visualizing the colored product which is usually the consequence of the immunoassay reactions. Glass fiber fleeces have been used for filtration but this simply adds another layer to the device. The difficulties arise because of the problems of accurately placing several layers of thin flexible strips in proper registry one on the other while at the same time retaining the sample placement zones, reaction zones and other areas of the strips in proper relationship with each other. The problems are further complicated by the difficulties of placing the completed device in or on a proper platform which is often a hollow casing with separable upper and lower members including fixed pillars and slots to prevent the device from moving and to retain defined areas of the device in proper position relative to viewing windows and other openings in the casing.

Immunoassay instruments such as the foregoing when employed to detect analytes in whole blood utilize labels on the antibodies to the analytes which give rise to a detectable product. In the usual case a labelled detector antibody reacts with one epitope on the analyte and a capture antibody reacts with another epitope on the analyte. Typically, the product is visibly detectable because it is colored. In some constructions, the color is apparent to the naked eye. In more sophisticated devices, the concentration of the analyte may be determined by measuring the intensity of the produced color with a suitable instrument. In both instances, the presence of red blood cells in the color development area interferes with proper visualization of the color because of the intense hue of the cells.

Much effort has been expended to prevent such interference. As a result, all devices proposed for analysis of whole blood include some type of filter to remove the red blood cells to produce serum or plasma which does not mar the visibility of the color which is produced. A glass fiber fleece issued in the device of United States Patent 4,477,575 to filter the red blood cells. Other patents describe the use of paper or plastic filters.

JP-A-60 94 718 discloses an assay in which a sample of whole blood is applied to a device. The membrane consists of glass fibres coated with silica gel. Antibody coated with gold colloid is used as detector complex. The developing velocities of the liquid and the cell portions are different and the result of the test may therefore be observed by the naked eye.

EP-A-505 636 discloses an immunoassay device made from for instance nitrocellulose. The derice strip contains a constriction which functions as flow control means and sample application zone, detector zone with mobile labelled antibody and capture zone.

### BRIEF SUMMARY OF THE INVENTION

This invention provides an assay in which all of the reactions necessary to determine the presence of an analyte in whole blood without interference by red blood cells take place on one or at most two membrane devices assembled by easy manufacturing techniques. The device used in the assay may be contained in a very simple casing.

Although the invention is not so limited, for convenience, it will be described as utilized for the detection of cardiac analytes in whole blood. It may additionally be employed for detecting and/or measuring a wide variety of analytes in blood, such as: drugs, including therapeutic drugs and drugs of abuse; hormones, vitamins, proteins, including antibodies of all classes, peptides; steroids; bacteria; fungi; viruses; parasites: components or products of bacteria, fungi, viruses, or parasites: allergens of all types: products or components or normal or malignant cells; etc. As particular examples. there may be mentioned digoxin, hCG; insulin; theophylline; luteinizing hormone; thyroid stimulating hormone; organisms causing or associated with various disease states, such as streptococcus pyrogens (group A), Herpes Simples I and II, cytomegalovirus, Chlamydia, rubella antibody, etc.

An important feature of this invention is the geometry of the device used in the assay which is designed to provide a stream from which substantially all of the red blood cells have been separated. As a result of the geometric design of the device, a fixed volume of plasma is in contact with a preselected area of the device for a longer period of time, thus providing sufficient contact time for binding reactions to take place. Said fixed volume is determined by the volumetric capacity of the membrane.

### BRIEF DESCRIPTION OF THE DRAWINGS

This invention will be best understood when considered in conjunction with the following description which, together with the drawings, form part of this specification wherein:
Fig. 1 is a plan view of a unilayer device used in the assay of the invention.
Fig. 2 is a plan view of a device in which a top long membrane is unfolded and rotated 180° with respect to another shorter membrane over which it is superimposed in fluid contact
Fig. 3 is a plan view of a product showing four devices in the same unitary structure.
Fig. 4 is an exploded view of an alternate product showing a bilayer device on a platform with upper and lower members.
Fig. 5a shows a section through a device housing corresponding to the current Figure 4.
Fig. 5b shows a plan view of the upper membrane for use in Figure. 5a.
Fig. 5c shows a plan view of the lower membrane for use in Figure 5a.
Fig. 6a shows a section through device housing avoiding the use of a hole in the upper membrane shown in Figure 4.
Fig. 6b shows a plan view of the upper membrane for use in Figure 6a.
Fig. 6c shows a plan view of the lower membrane for use in Figure 6a.
Fig. 7 shows the single membrane device used in Example 2. The parts are identified by name for easy understanding.
Fig. 8 shows the device used in Example 3. The parts are similarly identified.
Fig. 9 shows the device used in Example 4 similarly labeled.

### DETAILED DESCRIPTION OF THE INVENTION

In this description of the invention, the device is the membrane structure employed to chromatographically retard the flow of red blood cells in blood applied to an application zone thereof thereby to transform the whole blood into a stream with a plasma front followed by a front of the slower moving red blood cells. The device is particularly adapted and well suited for the reception and diagnostic processing of small quantities of fluid sample, such as droplets of whole blood received from a finger prick procedure. The membrane structure also serves as a substrate for the diagnostic reactions. The casing is the carrier, holder or platform for the device.

A major contributing factor to the invention described and claimed herein is the discovery that cellular membranes constructed, preferably, of nitrocellulose will preferentially retard or inhibit the flow of red blood cells moving along the membrane, thereby forming a stream presenting a red blood cell front and downstream thereof a plasma front. More specifically, the red blood cells are not filtered from the whole blood but are separated chromatographically from the plasma which, in the selected membrane substrate, flows faster than the red blood cells.

Another feature of the invention is that the plasma segment or section of the stream, which is substantially free of red blood cells, is manipulated so that it flows slowly through the area where certain of the diagnostically useful reactions take place. This feature will be explained in more detail hereinafter.

Devices for use in the invention are designed with a sample application zone to which the blood sample is applied, and downstream of the sample application zone, a flow zone, such as, for example an elongated reaction zone including a detector zone upstream of a capture zone. In this respect, a device used in the invention is not unlike previously known devices such as those described in the patents identified above. The critical features on all such devices is that an analyte, for example myoglobin or myosin light chain in the blood, reacts with a colored, mobile labelled antibody to the analyte in the reaction zone to form an antigen/antibody complex. This complex is soluble or suspended in the carrier liquid and moves down the membrane to react with a capture antibody to form a visible antibody/antigen/antibody reaction product, wherein the antigen is the analyte. All such devices when employed with whole blood to achieve diagnosis by the production of color product employ some type of filter to stop the flow of red blood cells and separate them from the areas where the useful diagnostic reactions take place to avoid interference with color formation and interpretation. Typically, the filter is a glass fiber, plastic, or cellulose paper filter, which removes the red blood cells as the blood moves longitudinally from the point of application towards the reaction area.

In contrast to such previously known diagnostic constructions, the device used in this invention achieves a chromatographic separation where the red blood cells continue to flow but at a slower rate than the plasma or serum. Hence the red blood cells are separated from the plasma chromatographically rather than by filtration.

It has been surprisingly discovered that whole blood, as it moves along a membrane such as a nitrocellulose membrane, is separated chromatographically into a relatively fast moving plasma stream and a relatively slow moving red blood cell stream. As a result, the red blood cells become separated from the plasma. In an assay of the invention, the initial immunological reactions may take place even before the plasma front of the stream substantially free of red blood cells has been formed. The reaction continues in the separated plasma to provide diagnostically useful information.

During an ischemic event such as stable or unstable angina or myocardial infarction, a variety of different proteins characteristic of the event are released by cardiac tissue. These proteins which are referred to as analytes may be used diagnostically as described in U.S. Patents No. 5,290,678 and No. 5,604,105, the disclosures of which are incorporated herein by reference. Typical examples of analytes are myoglobin, creatinine kinase-MB (CK-MB), myosin light chain, troponin I, and troponin T. As described in these patents, the presence of these analytes in whole blood is determined by antigen/antibody reactions which take place in laminated structure in which the analyte first contacts labelled detector antibody which reacts with a first epitope on the analyte to form a labelled antibodylanalyte complex. The complex then contacts a capture antibody which reacts with another epitope on the analyte to form a visible labelled antibody/analyte/detector antibody reaction product. As described in the patents, detection of the presence of at least three analytes in more than normal amounts permits the physician to accurately characterize the cardiac event as stable or unstable angina or as a myocardial infarction. There are many other cardiac analytes which may be similarly employed. Devices with only one or two analytes are also available, but generally are not capable of giving as much information as devices adapted to three or more analytes.

A clear understanding of this invention will be facilitated by a description of some specific embodiments, The description will be followed by a discussion of some of the general considerations involved in designing products.

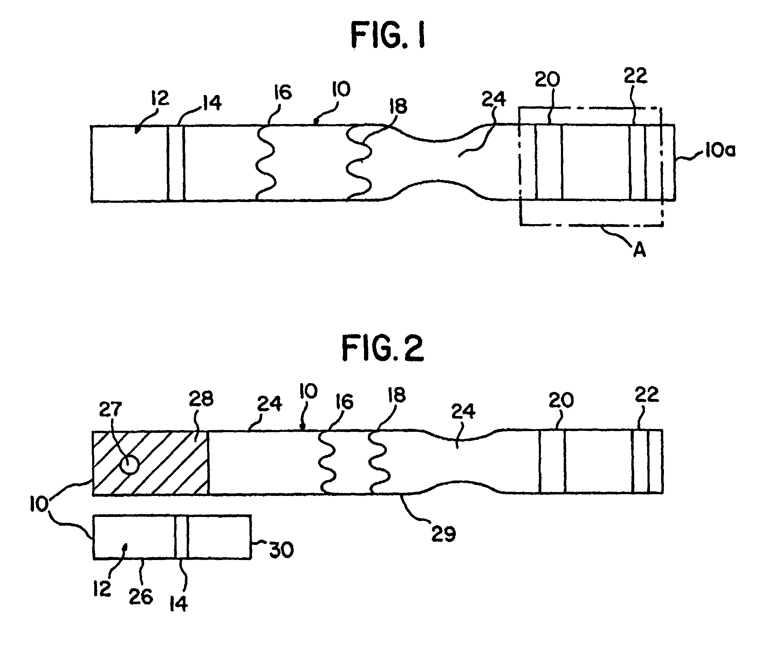

Figure 1 shows a typical device comprised of a membrane 10 configured as shown.

The membrane includes a sample application zone 12 to which one or more drops of whole blood may be applied. As noted earlier, it is a particular advantage of the invention that whole blood may be directly analyzed and that only small amounts of blood, e.g., about 8 to 20µl or even less are required. This amount of blood may be obtained and applied directly from a finger prick or from a dropper or pipette after withdrawal from a patient.

The blood moves downstream of the application zone 12 to a reaction zone which includes a detector zone 14 containing a labelled detector antibody reactive with an epitope on the analyte to produce a labelled antibody/analyte complex. This reaction is well known and need not to be described in detail.

Any of a variety of labels available to the skilled artisan may be utilized. Metal and enzyme labels are preferred. Metal labels are especially preferred due to their remarkable sensitivity. Amongst the metals, gold is most preferred principally because it is so widely employed for this type of reaction and its characteristics are so well understood. Additionally, a gold signal can be enhanced to become readily visible by the use of a soluble silver salt and a reducing agent in accordance with known procedures. The gold label acts as a catalyst to reduce the silver salt to metallic silver, which deposits as a visible product. A typical reactive pair is silver lactate, which serves as the source of reducible silver ions, and hydroquinine as a reducing agent. The metallic silver forms a readily discernible black deposit around each particle of gold.

There may be a preincubation zone starting in the application zone and continuing to the detector zone although it is not a necessary feature of the invention. The preincubation zone is employed to remove products present in the blood which may interfere with the desired reactions or make them difficult to detect. A typical interferant is the isoform of creatine kinase, CK-MM. Antibodies to the isoform CK-MB may cross react with CK-MM and give false readings. This can be avoided by providing sufficient immobilized antibody to CK-MM in a preincubation zone so that all of the CK-MM is removed before the moving sample reaches the detection zone.

The device of Figure 1 may utilize one or a plurality labelled detector antibodies. When several labelled detectors are employed care must be exercised to avoid interfering reactions. It is often best that the antibodies be arranged in one or more detection zones to react with their specific analytes.

The labelled detector antibody is mobile, i.e., it is movably bound to the membrane 10 in the detector zone 14 so that the labelled antibody/analyte complex, once formed, is free to move downstream. Of course, antibodies employed in this reaction or in reactions subsequently to be described may be either monoclonal or polyclonal. A large number of such antibodies are well known and readily available or can be prepared by standard procedures.

As the blood moves downstream away from the application zone 12 the red blood cells move more slowly than the plasma, and form a front 16. A proportion of the plasma component of the original blood sample continues downstream as a plasma segment the front of which is indicated in Fig. 1 at 18. Ultimately, and as indicated above, substantially all of the red blood cells are confined to an area encompassing the application zone 12 and the red blood cell front 16. If a labelled antibody/analyte complex has formed it will be dissolved in the plasma and carried towards the capture zone 20. In the capture zone, the complex will contact a capture antibody, which will react with the complex to form a detectable labelled antibodylanalytelantibody reaction product.

It will be noted that the first reaction of the labelled antibody and analyte may take place in the whole blood stream in the presence of the red blood cells. For proper analysis of the analytes in the blood sample, it is not necessary to know at this point that a detector antibody has reacted with an analyte. The red blood cells are not interferants. If desired, however, the complex may be formed downstream of the red blood cell front 16 by appropriate placement of the labelled antibody, but this may unnecessarily increase the length of the device.

If the label is a sol such as gold, a visible line will form, which as suggested above may be the gold product or may be reduced silver. If the label is an enzyme such as horseradish peroxidase, reaction may be detected by the addition of hydrogen peroxide and a dye such as ortho phenylenediamine in accordance with standard procedures.

The device of Fig. 1 includes a control zone 22, which may contain a product that reacts with any substance normally present in blood and in plasma to produce a visible product. The use of a control reaction is optional, but is preferred so that the operator will know that sufficient blood has been applied in the application zone 12 and that the diagnostic reactions have had the opportunity to take place.

Those skilled in the art will recognize that the reactions described will each have characteristic reaction kinetics and each will require a period of time for the reaction to be completed. To insure that there will be sufficient time for completion of the reaction with the capture antibody and production of sufficient readily visible product, the device is configured so that the flow rate of the plasma stream is retarded and there is more time for reaction of the labelled antibody/analyte complex with the capture antibody.

In general, modulation or control of the flow of the fluid sample and its components may be achieved by different means. For example, the membrane may be configured so that fluid flow (i.e., flow of the plasma) may be elongated to extend to the end of the membrane. Another way in which flow may be controlled is by constricting the channels, either in size or in number, through which such flow may take place. A third way to control flow is to apply a predetermined volume of sample to the membrane, so that the amount of such sample corresponds to the volume that will fill only the channels available in the membrane for such fluid flow.

Accordingly, the device may include a flow modulation or control means associated with the membrane or strips, which may comprise, for example, one or more restrictions in the width of the membrane, or the placement of one or more baffles or like flow barriers, to inhibit plasma flow. Such baffles may be formed by compressions in the membrane which would eliminate porosity and, consequently, would inhibit or prevent flow therethrough.

Flow modulation or restriction may also be accomplished by elongating the plasma stream so that the distance a fixed volume of plasma must flow before it reaches a capture antibody increases. The ultimate result is that the total volume of plasma remains in the capture zone 20 for a longer period of time. The volumetric capacity of the strip between capture zone 20 and end of the strip 10a determines the volume of plasma that flows through the capture region 20. That is flow stops when the strip is totally saturated. Furthermore, this also prevents further flow of red cells and prevents them from obscuring the capture region.

Another function of the elongation is to provide a greater separation between the red blood cell front and the plasma front 18 for a minimum plasma volume. This is important to minimize the possibility of the red blood cells migrating into the capture zone 20 and confusing the results. There are several means of accomplishing the elongation of the route the plasma segment must follow. One such means is shown in Fig. 1 which shows an indentation of the sides of the membrane to form a neck 24. The neck may be formed by cutting the edges of the membrane. However, such cutting should be accomplished, e.g., by a laser device, as conventional means may compress the capillary space, i.e., pores, along the edges of the channels in the membrane, and may thereby interfere with fluid flow. It may also be formed by compressing the membrane to form a compressed section of the desired shape through which the plasma is unable to flow. The same result may also be achieved by impregnating the membrane with wax or another inert substance to also form the desired configuration through which the plasma will not flow.

Still other alternatives include compressing a series of round, square or elongated designs across the membrane in the area shown as a neck 24 in Fig. 1. If this alternative is chosen, there is no indentation of the membrane, but the compressed segments impede the flow of the plasma in a manner analogous to that in which rocks impede the flow of water in a river.

Fig. 2 illustrates an embodiment of the invention in which the application zone 12 and detection zone 14 are in a lower membrane layer 26 under the upstream end of a upper membrane layer 29. The advantage of this design is that the length of the device and the flow time through the device are both decreased. This is accomplished while retaining the essential flow of the sample through the device and without loss of the chromatographic effect. Hence, a larger volume of blood can be analyzed in a shorter period of time. An alternate structure would be a locally increasing width of the membrane. However, this is not preferred since it alters the flow characteristics by forming stagnation regions in which plasma may collect but not flow.

In the device of Fig. 2, the application zone 12 is the section of the lower layer 26 under the through hole 27 through which the blood is applied before flowing downstream in the lower layer 26 through detection zone 14 and into the upper layer 29. The area around the through hole 27 in the application zone 12 of the upper layer 29 is a blocked area 28 formed for example by compression or with wax. The purpose of the blocking is to prevent the blood from flowing downstream in the upper layer 29 and forming a channel for the blood to flow into the detection zone 14 of the lower layer 26.

The whole blood flows through the detection zone 14 where the analyte, if present, contacts and reacts with a labelled detection antibody and then into the upper member 29. The operation of the device of Fig. 2 is thereafter the same as the device of Fig. 1. There is a red blood cell front 16, a plasma stream with front 18, a control zone 22 and a neck 24.

It will be noted that in the device of Fig. 2, the downstream end 30 of lower member 26 overlaps the blocked area 28 of upper member 29 so as to form a flow path for the blood. Blocked area 28 may consist of a wax or other aqueous liquid impervious coating to control liquid flow. The ultimate effect of the arrangement shown in Fig. 2 is to form a flow channel which forces the blood to flow from the application zone 12 through the detection zone 14 and towards the capture zone 20. The flow is still essentially along the membranes, thus maintaining the chromatographic effect.

The position of the layers shown in Fig. 2 may be inverted, in which case through hole 27 would not be required. The direction of flow is still substantially along the membranes.

The effect of the overlapping membrane construction is to maximize the amount of blood that the device can utilize without making the test duration excessive while still retaining essentially longitudinal flow.

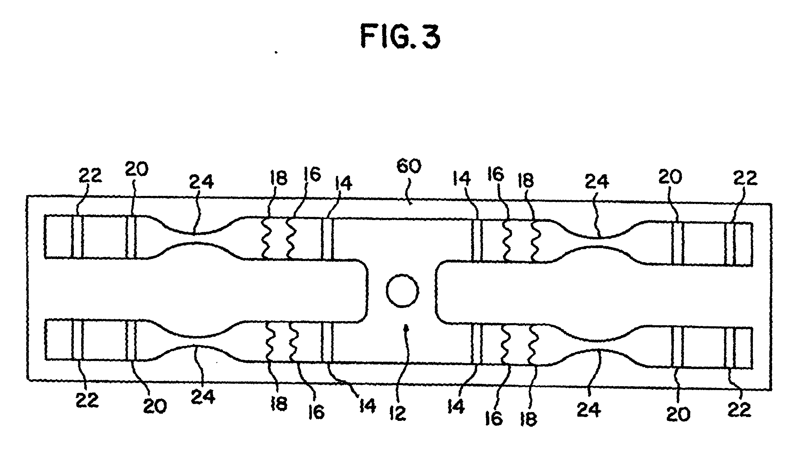

Fig. 3 illustrates an embodiment in which four devices are on the same substrate 60. All of the devices share application zone 12. Blood added at application zone 12 flows through each device and reacts in the same manner as previously described.

The multiple device embodiment may be printed on one sheet of membrane material by forming the lines defining each device by compression or heat or by printing the lines on the membrane sheet with wax or another material which will form the proper flow channels in each device. Alternatively, the multiple device can be separately formed by cutting it from a membrane sheet and adhering it to an inert substrate such as a plastic sheet with no capillary attraction for the flowing blood or plasma. In fact, nitrocellulose membranes with polyester backings are commercially available and presently preferred for producing the various devices.

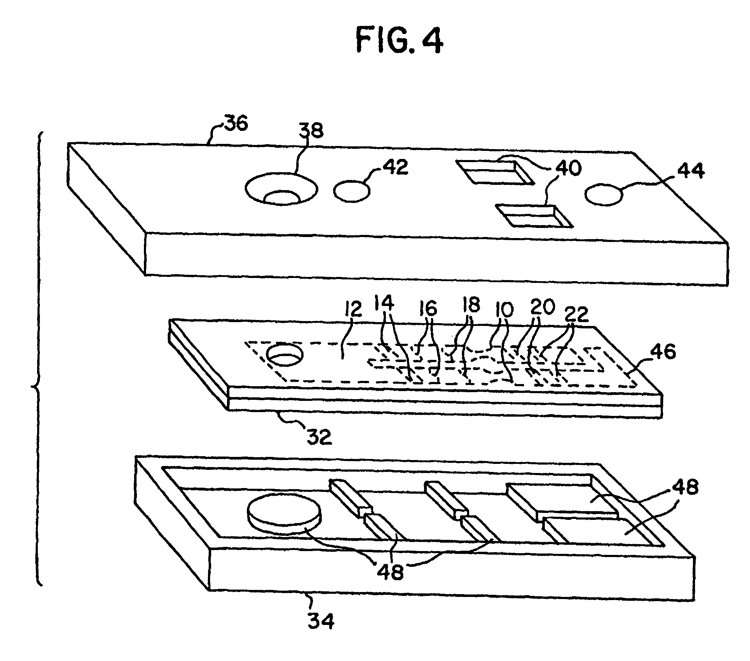

Other configurations of the multiple device embodiment will be apparent to those skilled in the art. For example, the devices can be set in a side by side configuration as shown in Fig. 4.

Fig. 4 illustrates an embodiment in which two adjacent devices are supported in a casing 30 having a cover member, 36, and a support member, 34. As shown, the product supports two devices, each with a detection zone 14. In Fig. 4, those numerals are identical to numerals in the other figures have the same meaning.

The figure is an exploded view showing two joined devices each designated as 10 backed with a polyester sheet 32 in the casing comprising a support member 34 and a cover member 36 for connection to the support member 34 to enclose the devices 10.

The cover member 36 includes a through hole 38 in registry with the application zone 12 for applying whole blood. It also includes view windows 40 in registry with the capture zones 20 present on each of the devices 10 and, if present, control zones 22, to permit the operator to view the results.

The cover member 36 may optionally contain a sufficient sample indicator hole 42, which will allow the operator to determine if sufficient blood has been added to complete the test. It may also contain a test end signal view hole 44, which will advise the operator that sufficient time has passed so that the results can be read without danger of false negatives due to early reading. The same safety requirement can be fulfilled with a stop watch or other timing device.

If a test end signal is desired, a spot of dye can be placed at the end of a device if only one is used, or at the end of one of the devices if multiple devices are employed. In either event, the dye will be upstream of the view hole 40. It will be dissolved in the flowing plasma and carried to the view hole 44 to signal the operator that it is time to read the test.

More specifically, the region 46 of device 10 (Fig. 4) shows the configuration of a timing device which extends under the viewing window 44. A dye will be placed between the control zone 22 and the section of the extension 46 which is under the window 44. The appearance of the dye under the window 44 will signal the operator that it is time to read the results.

Members 48 support the device in the casing 30.

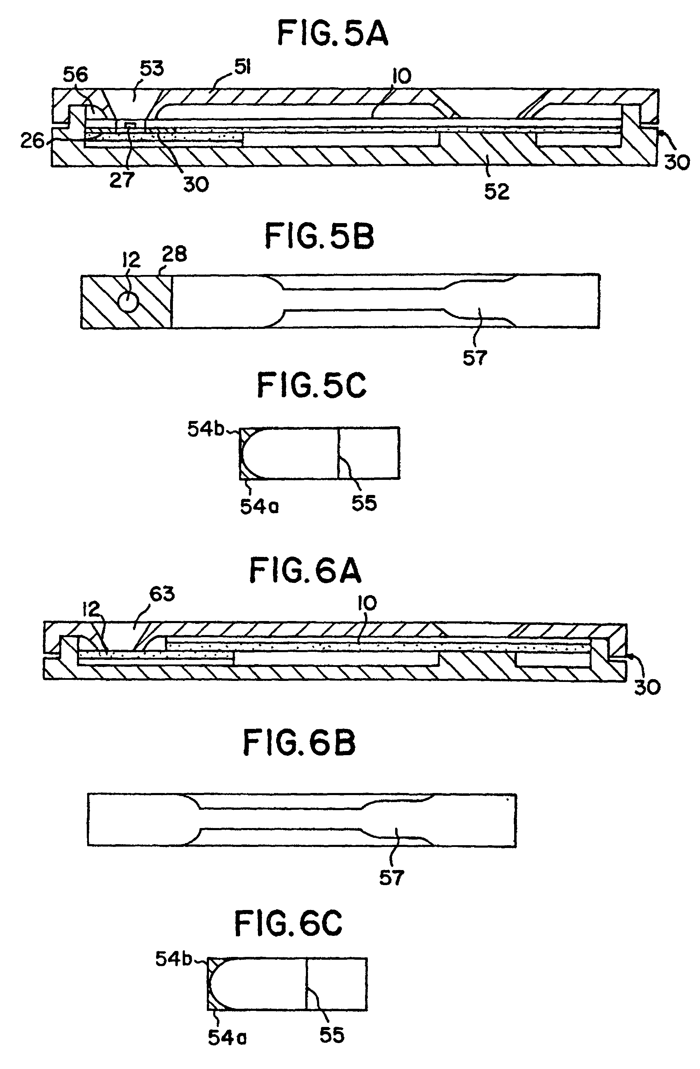

Fig. 5A shows a simplified cover member 51 and simplified lower support member 52. Aperture 53 provides access for blood to be applied through hole 27 in the upper membrane 29 and hence onto lower membrane 26. The dark region of the two membranes corresponds to the polyester backing, and the dotted region to the nitrocellulose. The hatched region 28 of upper membrane is a wax barrier to prevent blood wicking directly into upper membrane. The hatched regions 54a and 54b in lower membrane 12 merely prevent stagnation loss of blood in the comers. Wax line 55 serves to restrict the rate of flow of blood into the lower and upper membranes. Clearance region 56, surrounding aperture 53, prevents blood wicking along the capillary channels that may otherwise be formed between upper housing and the polyester surface of upper membrane. The width of capture region 57 is enlarged relative to throat region to provide improved visibility of the analytical result.

Fig. 6A is similar to the above but avoids the need to provide a hole in the upper membrane. The upper membrane 10 is made shorter so that aperture 63 connects blood application directly to the application zone 12 of the lower membrane 26. The other features are as described above and the numerals have the same meaning.

The operability of the test devices depends ultimately on separating the red blood cells from whole blood so as to permit substantially clear plasma containing the labelled antibody/analyte complex to reach the capture zone 20 so that a color change is detectable without interference from the strong color of the red blood cells. It was not, heretofore, appreciated that such a result could be realized without the use of separate vertical membranes to remove the red blood cells by filtration, in which the flow is through the thickness of the membrane.

A few simple observations will be sufficient to determine where the red blood cell front and clear plasma will appear on a cellular membrane of defined dimensions and pore size. Once that is known, the skilled artisan knowing the analyte which is being tested, the affinity for the analyte of the antibodies employed, and the kinetics of the reactions, will know where to place the antibody and the capture antibody as well as an appropriate length of the device. Even if these factors are not known, the optimum positions for the antibodies and the length of the plasma stream to provide the optimum signal can be readily determined empirically. All of this is well within the skill of the art.

The skilled artisan will recognize that any cellular membrane that chromatographically separates red blood cells and plasma from whole blood may be employed in this invention. However, nitrocellulose is preferred because it is readily available at reasonable cost. Nitrocellulose has been employed in chromatography and related fields for so many years that scientists and technicians are familiar with its properties.

Commercially available nitrocellulose can be readily formed into thin strips of any selected length and width.

The relevant nitrocellulose membranes may be characterized as sponge-like with a plurality of interconnected micropores of various sizes and dimensions giving rise to capillary forces within the membrane. This permits the biological fluid under investigation to move along the strip away from a sample application zone.

For the separation of plasma from red blood cells in the practice of this invention. the membrane material, geometry and dimensions are so selected that the desired reactions take place in preselected areas. These areas are selected on the basis of the relative speeds of the fronts of the red blood cell stream and the plasma stream. The kinetics of the desired reactions, the affinity of the antibodies for their respective epitopes, the size of the reacting particles and other factors are known or readily determined by conventional testing procedures.

While a variety of nitrocellulose membranes are available in various cell sizes, the presently preferred membranes are those which, if used as a filter, that is filtering particles from a liquid stream flowing vertically to the perpendicular surface of the membrane, will prevent the passage of particles larger than from 3 to 12 µm. In the practice of the invention, membranes with a pore size from about 5 to 12 µm. preferably 3 to 8 µm. are preferred. Some variation is possible. However, as the pore size decreases, the mobility of a fluid within the membrane decreases, thereby increasing the time required for diagnosis. If the pores are too large, the time of passage reduces with the result that the reactants are not in contact with each other for a sufficient period for the diagnostic reactions to occur, or to occur to such a limited extent that they do not provide the desired information.

Nitrocellulose membranes with supporting polyester or other films are commercially available. These are preferred for use in this invention since unsupported membranes tend to be quite fragile and susceptible to fracture. Moreover, the films are impervious to the flowing fluids so that substantially longitudinal flow channels can be formed by proper placement of the membranes within a housing. One such membrane is available to a variety of pore sizes from Gerbermembrane of Gerbershausen, Germany.

Procedures for the preparation of labelled antibodies for use in this invention are well known to the skilled artisan and need not be described in detail. The presently preferred labels are metal labels, particularly gold labels, the visibility and sensitivity of which can be enhanced using silver in accordance with known techniques. The preferred particle size for gold labelled antibodies used in the invention is from about 35 to 65 nm, although appreciable variation can be tolerated depending on well understood factors such as the concentration of the analyte and the affinity of the reactants.

The antibodies employed in this invention are prepared by standard techniques.
See for example, Falfre, Howe, Milstein et al., Nature Vol. 266, 7, 550-552, April 1977.

Procedures for fixing antibodies to substrates such as nitrocellulose are known and usable in producing the devices. Nitrocellulose is an avid binder for proteins. Hence, the immobile capture antibody need only be applied into the capture zone in a predetermined area. The labelled detector antibody may be movably affixed to the membrane by first saturating the detector zone 14 with another protein such as bovine serum albumin.

The optimum procedure for practicing this invention, which is applicable when antibodies with proper affinity for the analyte are available; when these antibodies can be effectively labelled with, for example a metal or enzyme label; when the reaction kinetics are favorable; and when the various reaction products move through the membrane at practical rates, is accomplished in four simple steps. These are:
1 ) Add a small amount of blood to the application zone,
2) Permit the blood to move into the detection zone where the analyte, if present, complexes with a mobile labelled antibody to the analyte to form a labelled antibody/analyte complex which will flow with the blood,
3) Allow the blood containing the complex to move through the membrane until a distinct red blood cell front 16 and a separate plasma front 18 have formed. The plasma contains dissolved labelled antibody/analyte complex, which it carries through the membrane towards the capture zone 20.
4) Permit the plasma stream substantially free of red blood cells to contact the capture antibody in the capture zone 20 for a sufficient period of time to produce a detectable labelled antibody/analyte/capture antibody reaction product.

There are, however, variations of this optimum process available if one or more of the above described conditions is not present. For example, it sometimes happens that the labelled antibody/analyte complex forms quite readily but does not combine with sufficient capture antibody to produce an easily detectable signal. This might happen if a sufficient number of the complexes do not contact capture antibodies or contact them in a configuration which is not optimal for forming a reaction product. Other possible problems are insufficient incubation time or low antibody affinity. These difficulties may be avoided by taking advantage of the avidin/streptabidin reaction or analogous reactions well known to the skilled artisan. The use of the biotin/avidin technique, of which the biotin/streptavidin reaction is an example, illustrates the versatility of the invention. It takes advantage of high affinity of streptavidin for biotin. More particularly, the use of biotin as the captured material and streptavidin as the capturing material confers the benefit of the faster and more positive interaction of these reagents for each other.

In one application of the process, two antibodies are removably fixed in the detection zone and streptavidin is in the capture zone. The detector antibody is labelled, preferably with a metal such as gold, and another antibody in the same zone is labelled with biotin. If the analyte for the antibodies is present in the plasma, a complex containing labelled detector antibody/analyte/biotin labelled detector antibody will form in the detection zone. The complex will move through the membrane in the plasma to the capture zone. Streptavidin is immobilized in the capture zone. When the complex reaches the streptavidin the latter binds to the biotin and concentrates the complex in a small area. As a result the signal becomes detectable. In this preferred variation of the biotin reaction, the biotin labelled antibodylanalyte/labelled detector antibody complex is made detectable by reaction of the biotin with streptavidin or analogous reactant in the capture zone.

This reaction scheme may be employed, for example, to detect myosin light chain (MLC). In this embodiment, the device is arranged with two antibodies to MLC in the detector zone. One antibody is labelled with gold, the other with biotin. If MLC is present in the blood being tested, the complex which forms will contain a gold labelled antibody, MLC and a biotin labelled antibody. This complex will move through the device to the capture zone and will react with immobilized streptavidin to produce a visible signal.

This invention has been described principally with reference to the detection of cardiac analytes. These include, for example, CKMB, MLC, myoglobin, glycogen phosphorylase, troponin T and troponin I. The devices and products may also be utilized as will be apparent to the skilled artisan to detect a number of other substances in whole blood. These include, for example, *H. pylori*, antibodies, HCG and hLH.

The test described and claimed herein in its various aspects is simple and flexible. The products are readily produced and do not require additional prior art features such as porous media waste sinks, and filter layers. A primary feature of the invention is that only low volumes of blood are required. Moreover, no wash liquid is employed.

The following non-limiting examples are given by way of illustration only.

### EXAMPLE 1

### SEPARATION OF RED BLOOD CELLS

Cellulose nitrate membranes (polyester supported, 3 µm and 8 µm nominal pore size from Gerbermembrane GmbH, Gerbershansen, Germany) were tested for the ability to retard the flow of red blood cells from whole blood subjected to a horizontal chromatography.

The membranes were cut to a length of 45 mm and a width of 6 mm. To an application area (7 mm x 6 mm) at one end, 15 µl of whole blood was applied. After the end of the chromatographic separation, the distance of the plasma front and of the red blood cell front from the origin was measured as follows:

| Membrane | Haematocrit | Red Blood Cell Front | Plasma Front |
|---|---|---|---|
| 3 µm Gerbermembrane | 36 | 10 mm | 22 mm |
| 3 µm Gerbermembrane | 39 | 10 mm | 21 mm |
| 3 µm Gerbermembrane | 41 | 10 mm | 21 mm |
| 3 µm Gerbermembrane | 47 | 10 mm | 19 mm |
| 3 µm Gerbermembrane | 51 | 10 mm | 19 mm |
| 3 µm Gerbermembrane | 55 | 11 mm | 18 mm |
| 8 µm Gerbermembrane | 36 | 14 mm | 27 mm |
| 8 µm Gerbermembrane | 39 | 14 mm | 26 mm |
| 8 µm Gerbermembrane | 41 | 14 mm | 24 mm |
| 8 µm Gerbermembrane | 47 | 14 mm | 22 mm |
| 8 µm Gerbermembrane | 51 | 15 mm | 23 mm |
| 8 µm Gerbermembrane | 55 | 16 mm | 23 mm |

### EXAMPLE 2

### DETECTION OF MYOGLOBIN IN WHOLE BLOOD

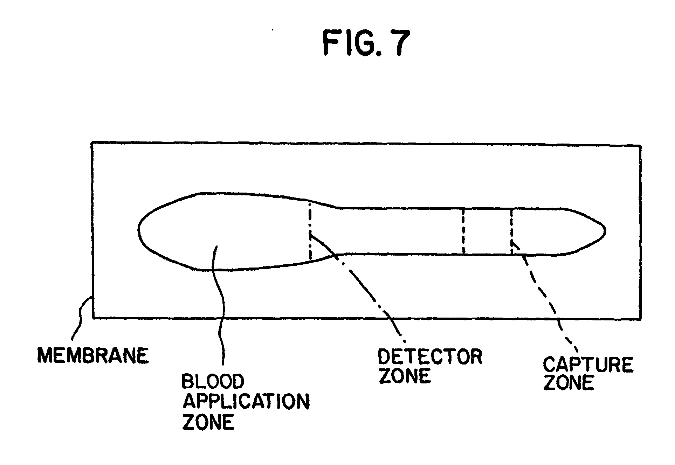

On a 20 mm x 10 mm membrane sheet (cellulose nitrate, 8 µm nominal pore size, Gerbermembrane) a contour shown in Figure 7 is prepared by a wax printing technique (as published in Laboratory News January 1996). Anti-myoglobin gold sol conjugates were prepared by British Biocell, Inc. (BBI), Cardiff, UK (40 nm gold sol, loaded with the antibody 2Mb-295 from Spectral Diagnostics, Toronto at a concentration of 1.5 µg/ml at an optical density (520 nm) of 1). To 10 ml of this solution 400 mg of bovine IgG (from Sigma-Aldrich GmbH, Deisenhofen, Germany) and 5 mg of octyl-D-glucopyranoside (from Fluka Chemie AG, Buchs, Switzerland) were added, and 0.8 µl of this mixture was applied to the detector zone. The capture line was prepared by impregnation with a solution of polyclonal rabbit anti-myoglobin antibodies from Spectral Diagnostics (lot number 95 APMO75C) at a concentration of 3 mg/ml in phosphate buffered saline, pH 7.3. to create the capture zone.

To the blood application zone, 4 µl of whole blood were added at the myoglobin concentrations shown below and the results (within 3 to 3.5 minutes) were as follows:

| Myoglobin Concentration | Signal at capture line |
|---|---|
| 50 ng/ml | weak signal |
| 200 ng/ml | clear signal |
| 500 ng/ml | strong signal |

### EXAMPLE 3

### SIMULTANEOUS DETECTION OF CK-MB AND MYOGLOBIN IN WHOLE BLOOD

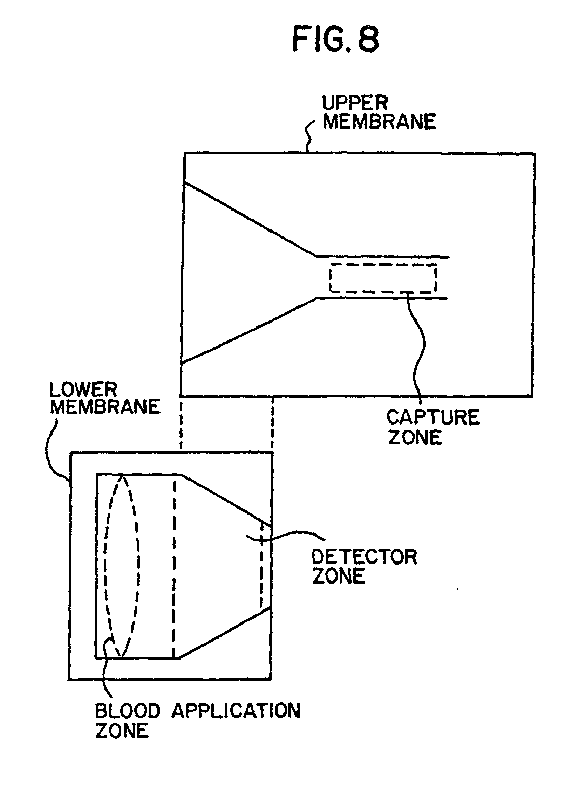

The configuration on appropriate membrane sheets illustrated in Fig. 8 was obtained by drawing on a 26 mm x 20 sheet (upper membrane) and a 10 mm x 20 mm sheet (lower membrane) the indicated lines with an 'edding 780' pen (from Edding AG, Ahrensburg, Germany). The dried ink forms a barrier for aqueous solutions, similar to the wax printing procedure in Example 2.

The capture zone of the upper membrane layer (polyester supported cellulose nitrate membrane, 8 µm nominal pore size from Gerbermembrane GmbH) contains a first line impregnated with a solution of Streptavidin (from Sigma-Aldrich GmbH, Deisenhofen. Germany) in water at a concentration of 30 mg/ml. The impregnation solution of the second line is a 3 mg/ml solution of rabbit-anti-myoglobin antibodies (Spectral Diagnostics, lot number 95 APM075C) in phosphate buffered saline. To increase the reproducibility of membrane wetting, indicated areas were impregnated with a mild detergent solution of 0.05% (w/w) of octyl-D-glucopyranoside (from Fluka AG. Switzerland) in water.

The detection zone of the lower membrane layer (polyester supported cellulose nitrate membrane, 3 µm nominal pore size, from Gerbermembrane GmbH) is impregnated with the following solutions:
a) Three µl of a gold sol conjugate solution.
This solution is prepared as follows (anti-CK-MB-and anti-myoglobin-gold sol conjugates were prepared by BBI, Cardiff, UK). To 900 µl of anti-CKMB gold sol conjugate (40 nm gold sol loaded with 22 µg/ml (at an OD of 10) of the antibody 5CKMB-6 from Spectral Diagnostics) 100 µl of anti myoglobin gold sol conjugate (40 nm gold sol loaded with 15 µg/ml (at an OD of 10) of the antibody 2MB-295 from Spectral Diagnostics) are added. Further 10 mg of octyl-D-glucopyranoside (from Fluka AG, Buchs, Switzerland) are added and mixed.
b) Two µl of a biotinylated antibody solution.
Two µl of a solution of 9.5 mg/ml biotinamidocaproate N-hydroxysuccinimide ester (from Sigma-Aldrich GmbH) in 1 ml of acetonitrile-water (1:1, v/v) were added to 1 ml of a solution containing 2 mg/ml of the antibody rCKMB-28 (from Spectral Diagnostics) in 75 mM potassium phosphate pH 8.5, mixed and incubated for 2 hours followed by the addition of 20 µl of a solution of 500 mM DL-lysinemonohydrochloride (from Fluka AG) and 100 mM potassium phosphate, final pH 8.5, and incubated for 0.5 hours. The solution was dialyzed over night against 5 mM potassium phosphate, 10 mM sodium chloride, pH 7. The antibody solution was diluted with water to a concentration of 35 µg/ml and to 1 ml of this diluted solution 10 mg of Crotein C and 3 mg of octyl-D-glucopyranoside were added to give the biotinylated antibody solution.
c) Three-point-five µl of a buffer solution of 75 nM HEPES pH 6.8 and 0.05% octyl-D-glucopyranoside.
Twenty-five µl of heparinized whole blood containing myoglobin (Spectral Diagnostics, lot number 3-10238) or rCK-MB (Spectral Diagnostics, lot number 3-24363G) at indicated concentrations were applied to the application zone and the results were as follows (within 9 to 12 min.):

| Trial | rCK-MB ng/ml | Myoglobin ng/ml | Signal*)first line | Signal second line |
|---|---|---|---|---|
| 1 | - | 50 | - | + |
| 2 | 8 | 200 | + | +++ |
| 3 | 50 | 200 | ++ | +++ |
| 4 | 200 | 500 | +++ | +++ |

| | | | | |
|---|---|---|---|---|
| *) "-" no visible signal line "+++" strong visible signal line | | | | |

### EXAMPLE 4

### DETECTION OF TROPONIN I IN WHOLE BLOOD

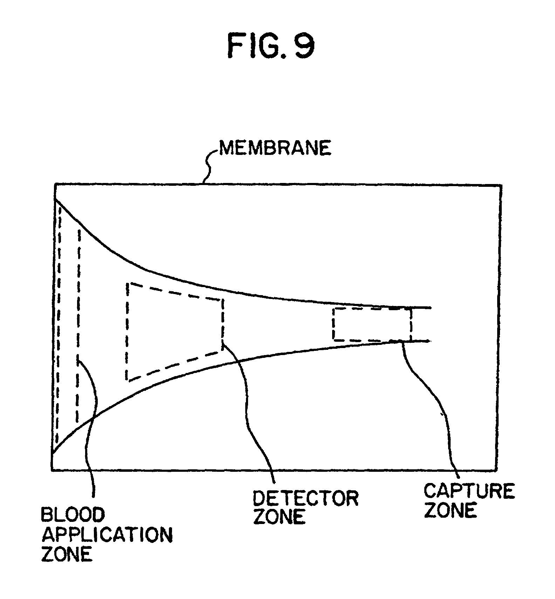

In Figure 9 is illustrated the design drawn with a Staedtler Lumocolor 313 pen (from Staedtler, Nuernberg, Germany) on a 30 mm x 35 mm sheet of membrane (cellulose nitrate, 3 µm nominal pore size, Gerbermembrane).

In the capture zone a line is impregnated with a solution of 13 mg/ml of streptavidin (poly) from Microcoat GmbH. Penzberg, Germany. After drying, the membrane is impregnated with a 0.5% aqueous solution of Crotein C (from Croda GmbH) in the detector and application zone to prevent unspecific binding. The detector zone was then impregnated with:
a) Five µl of a gold sol conjugate solution:
   To 90 µl of an anti-TN-I gold sol conjugate (60 nm gold sol loaded with 10 µg/ml (at an OD of 10) of the antibody 2I-14 from Spectral Diagnostics) of an OD of 30, 10 µl of a 250 mM sodium succinate buffer pH 5.5 is added and mixed to give the gold/sol conjugate solution.
b) Two µl of a biotinylated antibody solution:
   The biotinylation is done as in Example 3 but with the anti-TN-I antibody 8I-7 from Spectral Diagnostics. To 50 µl of this biotinylated antibody solution at a concentration of 600 µg/ml, 50 µl of a solution of 0.6% octyl-D-glucopyranoside and 200 mM MES, pH 5.5 were added and mixed to give the biotinylated antibody solution.

Thirty-five µl of heparinized whole blood containing TN-I at indicated concentrations were applied to the application zone and the results were as follows (within 9 to 12 min.):

| TNI ng/ml | signal *at capture line |
|---|---|
| - | - |
| 0.5 | + |
| 1 | ++ |
| 2 | ++ |

| | |
|---|---|
| *) "-" no visible signal "+++" strong visible signal | |

## Claims

1. A method of detecting the presence of an analyte in whole blood, comprising:
a. depositing a small volume of said whole blood on a device, said device comprising a cellular membrane, said membrane having a sample application zone to which the blood sample is applied and, downstream of the sample application zone, an elongated flow zone, including a detector zone and a capture zone, the detector zone containing a mobile labelled detector antibody which will react to form a mobile antibody/analyte complex which will move downstream, the membrane causing red blood cells to separate chromatographically from the plasma to cause the formation of a plasma front moving towards the capture zone and having a red blood cell front upstream thereof; the major portion of any complex being dissolved in the section of the stream between the two fronts which is substantially free of red blood cells, the capture zone containing a reactant which will react with the complex to form a detectable product; and wherein said cellular membrane includes a flow control means located downstream of said sample application zone which contributes to the separation of red blood cells from plasma; and
b. examining the capture zone of said membrane for the presence of a reaction, whereby said reaction signals the presence of the analyte sought to be detected.

## Patentansprüche

1. Verfahren zur Feststellung der Anwesenheit eines Analyten in Vollblut, welches umfasst,
a. dass ein kleines Volumen des Vollbluts auf eine Vorrichtung aufgebracht wird, wobei die Vorrichtung eine celluläre Membran umfasst, welche eine Probenaufgabezone aufweist, auf welche die Blutprobe aufgegeben wird, sowie stromabwärts von der Probenaufgabezone eine langgestreckte Strömungszone, welche eine Detektorzone und eine Einfangzone beinhaltet, wobei die Detektorzone einen mobilen, markierten Detektor-Antikörper enthält, welcher unter Bildung eines mobilen Antikörper/Analyten-Komplexes reagiert, welcher sich stromabwärts bewegt, wobei die Membran bewirkt, dass sich die roten Blutzellen chromatographisch vom Plasma unter Bildung einer Plasmafront, welche sich in Richtung auf die Einfangzone bewegt, sowie stromaufwärts davon einer roten Blutzellenfront trennen; wobei die Hauptmenge eines gelösten Komplexes im Abschnitt des Stromes zwischen den beiden Fronten vorliegt, welcher im Wesentlichen frei von roten Blutzellen ist, wobei die Einfangzone einen Reaktanten enthält, welcher mit dem Komplex unter Bildung eines detektierbaren Produktes reagiert; und wobei die celluläre Membran stromabwärts von der Probenaufgabezone angeordnete Einrichtungen zur Strömungskontrolle bzw. Strömungsbeeinflussung beinhaltet, welche zur Trennung der roten Blutzellen vom Plasma beitragen; und
b. dass die Einfangzone der Membran auf das Vorliegen einer Reaktion, welche die Anwesenheit des zu detektierenden Analyten signalisiert, untersucht wird.

## Revendications

1. Procédé pour la détection de la présence d'un analyte dans du sang total, comprenant:
a. la déposition d'un petit volume dudit sang total sur un dispositif, ledit dispositif comprenant une membrane cellulaire, ladite membrane ayant une zone d'application de l'échantillon à laquelle l'échantillon de sang est appliqué et, en aval de la zone d'application de l'échantillon, une zone rallongée de flux, comportant une zone de détection et une zone de capture, la zone de détection contenant un anticorps détecteur marqué mobile qui réagira pour former un complexe anticoips/analyte mobile qui se déplacera vers aval, la membrane provoquant la séparation chromatographique des érythrocytes du plasma pour provoquer la formation d'un front plasmatique se déplaçant vers la zone de capture et ayant un front d'érythrocytes en amont de celui-ci; la majeure partie de tout complexe étant dissoute dans la section de flux entre les deux fronts qui est essentiellement exempt d'érythrocytes, la zone de capture contenant un réactif qui réagira avec le complexe pour former un produit détectable; et dans lequel ladite membrane cellulaire inclut un moyen de contrôle du flux situé en aval de ladite zone d'application de l'échantillon qui contribue à la séparation des érythrocytes du plasma; et
b. l'examen de la zone de capture de ladite membrane pour la présence d'une réaction, par laquelle ladite réaction signale la présence de l'analyte que l'on cherche à détecter.
